# EUROPEAN PATENT APPLICATION

(11) **EP 1 269 991 A2**
(43) Date of publication of application: **02.01.2003**
(21) Application number: 02425243.9
(22) Date of filing: 18.04.2002
(51) Int. Cl.: A61K 7/48, A61K 31/00

(54) **Composition for topical use based on the ethylic ester of linoleic acid and on the triethyl ester of citric acid associated with suitable synergists**

(30) Priority: 20.06.2001 IT BS20010046
(71) Applicant: De Paoli Ambrosi, Gianfranco, 25087 Salo' (Brescia) (IT)
(72) Inventor: De Paoli Ambrosi, Gianfranco, 25087 Salo' (Brescia) (IT)
(74) Representative: Manzoni, Alessandro

(57) **Abstract**

The present invention relates to a composition for topical use, in particular aimed at skin care and at improving the aesthetical conditions of the same, characterised in that as active ingredient, it contains at least one of the active principles selected between the ethylic ester of linoleic acid and the triethyl ester of citric acid, which may be used both individually and in association.
When used individually, the ethylic ester of linoleic acid, or ethyl linoleate, can be contained in the composition in a quantity in weight preferably between 1.0 and 20%. When used individually, the triethyl ester of citric acid, or triethyl citrate, can be contained in the composition in a quantity in weight preferably between 0.50 and 30%. When used in association, ethyl linoleate and triethyl citrate can be contained in the composition in a quantity in weight preferably equal, comprised between 1.00 and 40% each.

## Description

### FIELD OF APPLICATION

The present invention relates to a new product based on ethylic esters of essential fatty acids, and on ethylic esters of mono, di-, tricarboxylic acids usable individually or in association in formulations for pharmaceutical and/or cosmetic use, for example in the treatment of acne and cutaneous seborrhoea.

### PRIOR ART

Acne affects a large number of people, and traces a cutaneous pathological frame characterised by a morphological and functional alteration of the piliferous-sebaceous unit with the appearance of white-heads (closed comedos), blackheads (open comedos), papules and, in the most serious forms, pustules, nodules, cysts and scars.

Acne affects about 80% of the population aged between 12 and 30, and especially in women it can continue even in old age.

The acne etiopathogenesis is strictly related to:
- the increased production of sebum (seborrhoea)
- the anomalous keratinization of the piliferous-sebaceous duct
- the bacterial colonization

Seborrhoea is the consequence of an exasperate response of the sebaceous gland to the action of androgenic hormones, more in detail to the action of dihydro-sterone obtained by the reduction of testosterone by the enzyme 5-α reductase.

The anomalous keratinization of the piliferous-sebaceous duct is directly responsible for the formation of the homy plug, the cause for the formation of the micro-comedo and of the subsequent acne lesions.

Due to the increased production of sebum and before the formation of the micro-comedo, there occurs an abnormal growth of the skin saprophyte bacteria, such as Propionil Bacterium Acnes.

Due to the release of lithic enzymes (protease and lipase), the latter is capable of causing the destruction of the sebaceous gland proteic structures and of hydrolysing the triglycerides normally contained in the sebum (which, among the other things, is produced in higher amounts due to the action of dihydro-sterone), thereby releasing fatty acids and glycerol.

The fatty acids thus released are characterised by the comedogenic action, and are thus oxidised, with the formation of chemical entities having a pro-inflammatory action.

The therapy implemented so far for the pharmacological treatment of acne or cosmetic treatment of seborrhoea has basically been based on the action of keratolytic and/or antibiotic substances, as well as other active principles, such as retinoids.

Among such substances we may mention, for example, salicylic acid, tartaric acid, glycolic acid, resorcin, phenol, etc. All of them are capable of carrying out their action aimed at the clinical Improvement of the acne history through a keratolytic mechanism.

Among the antibiotic substances used so far to keep the Proplonilbacterium Acnes growth under control, we may mention clindamycin, minocycline, erythromycin, metronidazole, etc.

Other active principles used for the treatment of acne are retinal acid, especially in the trans form, which is effective but characterised by a high toxicity, photo-toxicity, teratogenicity.

### OBJECT AND SUMMARY OF THE INVENTION

The object of the invention is to provide a product having a high efficacy and excellent cutaneous tolerability by an innovative mechanism of action capable of reducing seborrhoea, controlling the growth of the saprophyte bacterium Propionilbacterium Acnes and inhibiting the hyper-keratinization of the piliferous-sebaceous duct.

This object is achieved by a composition containing, as active ingredient, at least one of the substances including the ethylic ester of linoleic acid and the triethyl ester of citric acid, which may be used both individually and in association.

When used individually, the ethylic ester of linoleic acid, or ethyl linoleate, can be contained in the composition in a quantity in weight ranging between 0.1 and 100%, preferably between 1.0 and 20% based on the final formulation.

When used individually, the triethyl ester of citric acid, or triethyl citrate, can be contained in the composition in a quantity in weight ranging between 0.1 and 100%, preferably between 0.50 and 30% based on the final formulation.

When used in association, ethyl linoleate and triethyl citrate can be contained in the composition in a quantity in weight ranging between 0.1 and 99.9% each, preferably in an equal amount between 1.00 and 40% each.

Moreover, the composition based on ethyl linoleate and/or triethyl citrate can contain various active ingredients, which for the sake of simplicity we shall call synergists.

Synergists can be selected among acetic acid, lactic acid, salicylic acid, tartaric acid, glycolic acid, clindamycin, erythromycin, metronidazole, amoxicyllin, triclosan, capryloyl glycine, azelaic acid, hydroxide zinc, chloride zinc, retinol, trans-retinoic acid, resorcin, gentamicin, meclocycline, phenol, ascorbic acid, tocopherol, lipoic acid, phosphatidylcoline, phosphatidylserine, clorexidine, irgasan, phospholipids in general, in all dextrorotatory and laevorotatory forms, racemic mixtures, cis forms, trans forms and relevant salts, esters and amides, and formulations with particular additives and excipients for external use.

Such synergists can be present in the composition - both Individually and in combination - in the number of two or more, along with ethyl linoleate and/or triethyl citrate.

Such synergists can be contained in variable quantities in weight, between 0.001 and 70%, preferably between 0.5 and 15%, based on the final formulation, when the proportions of ethyl linoleate and/or of triethyl citrate are from 0.05 to 90.5% in weight each.

### DETAILED DESCRIPTION OF THE INVENTION

The clinical efficacy and the safety of use of the preparation based on ethyl linoleate and/or triethyl citrate has been experimentally documented with evident and objective results.

The clinical efficacy and the safety of use are the consequence of an original mechanism of action characterised in that both ethyl linoleate and triethyl citrate behave as inert substances and are transformed into active principles when they come into contact with the skin.

Such transformation, from Inert substance to active principle, is a consequence of the hydrolysis that occurs due to specific cutaneous (esterase) or bacterial (lipase) enzymes, capable of releasing ethylic alcohol and, respectively, linoleic acid, diethyl citrate, mono-ethyl citrate and then, citric acid.

Such mechanism of action can be described more in detail with the aid of the following diagrams:
1. control of seborrhoea and hyper-keratinization:
   a. Ethyl linoleate is an inert ingredient hydrolysed by the bacterial lipases released by the Propionil Bacterium Acnes and/or by any other bacteria present onto the skin and/or by the esterase normally present on the skin, thereby causing the formation of ethylic acid and linoleic acid.
   b. Linoleic acid is capable of inhibiting the action of the enzyme 5-α reductase, responsible for the reduction of testosterone and dihydro-sterone, whose biological activity at the level of the sebaceous gland causes an increase of the production of sebum (seborrhoea).
   c. In the acne history, the hyper-keratinization of the piliferous-sebaceous duct is in strict relation with a drop of the levels of linoleic acid. The exogenous rate of this compound modifies the keratinization dynamics, intervening on one of the main causes of the acne evolution. 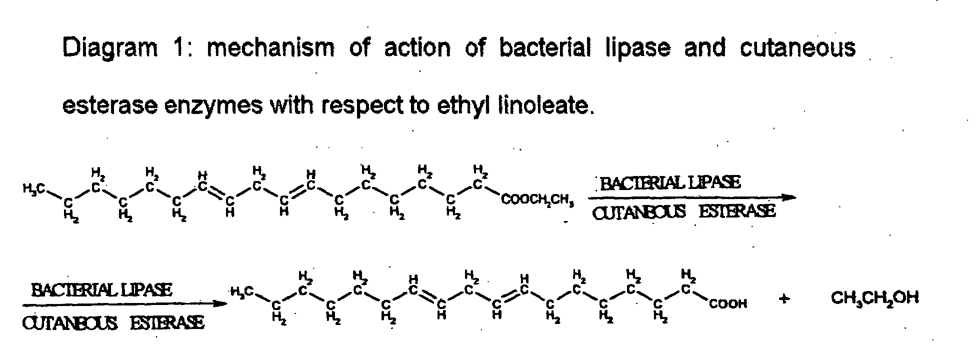 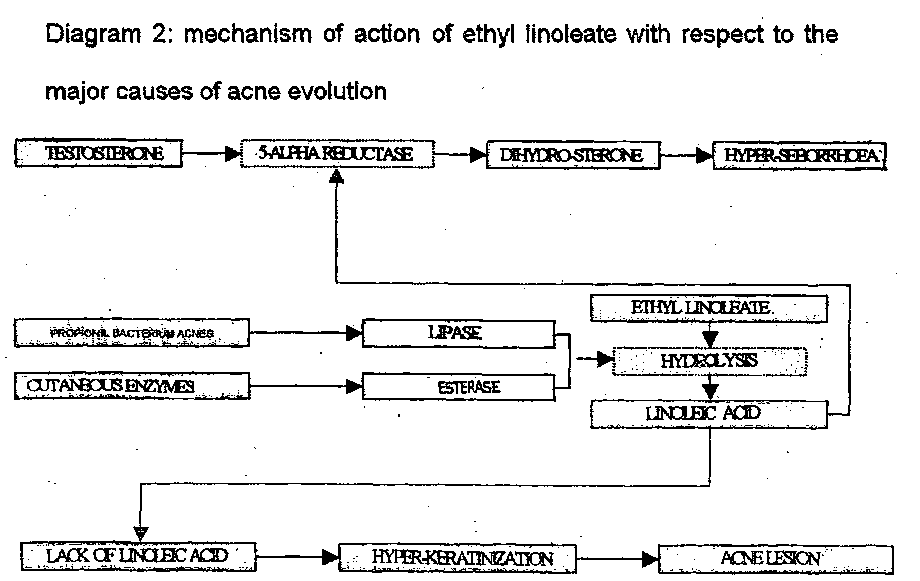
2. Control of bacterial growth
   a. Triethyl citrate is hydrolysed by the bacterial lipases produced by Propionil Bacterium Acnes and/or by cutaneous esterase, releasing in a differentiated manner the first carboxyl, the second one and finally, the third one. Each of the three carboxyls is characterised by different values of pKa, and thereby, acidity:
      i. first carboxyl pKa = 3.128
      ii. second carboxyl pKa = 4.761
      iii. third carboxyl pKa = 6.396

      The cutaneous pH is thus dynamically modulated towards such values of acidity as to inhibit the growth of the Propionilbacterium acnes and the consequent release of lipase capable of hydrolysing the triglycerides contained in the sebum, from whose hydrolysis the comedogenic fatty acids are released. Once oxidised, the latter are transformed into compounds provided with anti-inflammatory activity. Through such mechanism of action, as determined in diagrams 3 and 4, it is possible to trigger a virtuous circle capable of impeding the evolution of the major phases of the acne pathology. 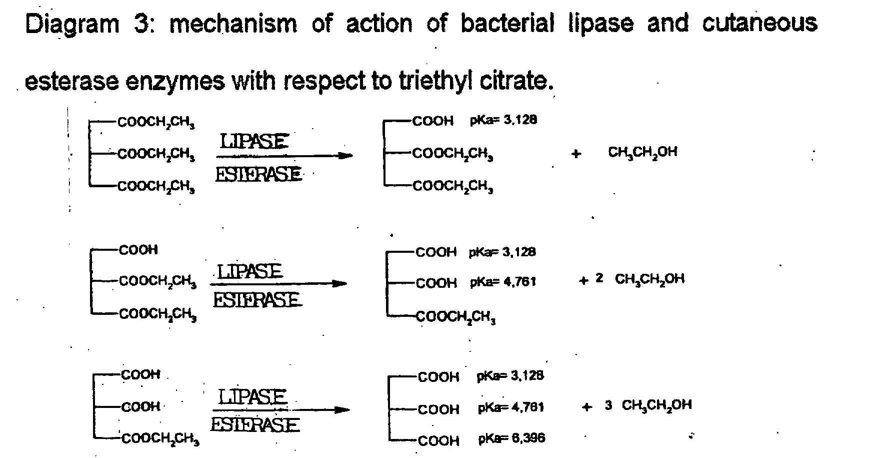 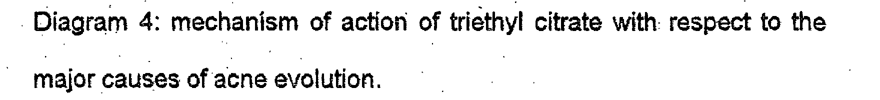 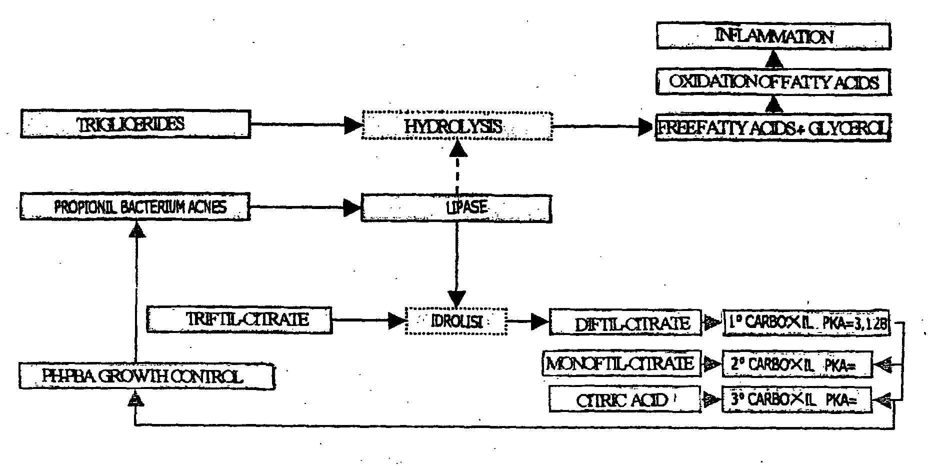
3. Higher affinity of bacterial lipase with respect to the hydrolysis of ethyl linoleate and of triethyl citrate rather than the sebum triglycerides.

Another important aspect of the present invention is that the cutaneous lipases have shown a higher affinity to hydrolyse ethyl linoleate and triethyl citrate rather than the triglycerides contained in the sebum.

This is very important to control the evolution of acne; in fact, the release of comedogenic fatty acids (obtained by the triglyceride hydrolysis), the consequent oxidation to compounds characterised by a pro-inflammatory activity, is one of the most crucial moments in the evolution of acne.

On the other hand, the release of linoleic acid and of citric acid obtained by the hydrolysis of the respective ethylic esters allows the pharmaceutical action previously described at 1 and 2, and ensures a virtuous circle that allows:
a. the control of hyper-seborrhoea;
b. the control of bacterial growth;
c. the control of the infra-infundibule hyper-keratinization;
d. the control of the amount of bacterial lipases produced;
e. preference, in any case, of the hydrolysis of ethylic esters of linoleic and citric acids rather than the sebum triglycerides;
f. consequent control of the amount of fatty acids released by the hydrolysis of triglycerides;
g. general control of the inflammatory process.

As known, acne is a chronic Inflammatory pathology of the piliferous-sebaceous unit in which 4 converging pathogenic moments occur: increased sebaceous secretion, anomaly of the superficial cutaneous bacterial flora, hyper-keratosis of the piliferous-sebaceous unit and action of the inflammation mediators.
within the scope of acne polymorphism, comedos can have a primary or secondary role with respect to papules and pustules. In the first case, the so-called comedo acne occurs. In any case, there exists a considerable correlation between the severity of acne and the number and size of comedos.

Recent studies show that comedos are lesions induced by the hyper-proliferation of infundibular cells.

The treatment of piliferous-sebaceous unit disorders needs a serious commitment in terms of skill, expertise, possibility of having effective products.

The prevention and removal of comedos and the control of skin susceptible to infection is a very important issue in cosmetology, which bases the treatments on skin cleansing, the use of topical antiseptics, the use of smoothing or slightly abrasive products, and lenitive products capable of attenuating the irritations caused by inflamed comedos. There is a wide range of face cleansing products, such as soaps or non-soap detergents with antiseptic action, or containing sulphur.

The use of cosmetic products, however, does not intervene on the cause of acne, bur rather they try to restore the acne skin to normality.

### EFFECTS OF THE INVENTION IN RELATION TO SAMPLE EXPERIMENTATION

Based on the present invention, clinical tests with the use of a sebum-meter have been carried out to evaluate the action of two anti-acne products.

This was carried out according to the following parameters:

### Object

The test allows evaluating whether the products subject to test are a valid aid in the treatment of acne and whether they can attenuate reddening caused by the presence of acneic centres of infection.

### Experimental sample

Five female volunteers with oily skin and suffering from acne, aged between 15 and 28

### Preparation of the samples

The samples must be applied as they are, based on their use features.

### Method of application of the samples

The samples must be evenly applied on specific areas of the face, according to the instructions reported in the description card supplied to the volunteer.

The lotion on the right side of the face.

The cream ion the left side of the face.

### Execution of the test

After recruiting the volunteer, the following instrumental evaluations are carried out:
- Basal measurement of sebum using a sebum-meter authorised according to CEE standards (SKIN LAB)
- Basal hydration using an instrument authorised according to CEE standard (SKIN LAB)
- Basal TEWL using an instrument authorised according to CEE standard (Tewameter) only on the left side of the face
- Acquisition of micro-pictures using a polarised-light telecamera, 200X. The micro-images are used to view the depth of the imperfections caused by acne and to highlight any improvements during treatment.
- Acquisition of macro-pictures using a Mini DV. The pictures are useful to define the general initial situation and to document any macroscopic improvements during the use of the products.
- The volunteers are provided with cards where they will record daily remarks regarding the cosmetic pleasantness of the products, and their functionality.

The volunteer is provided with a descriptive card regarding the various products subject to testing.

To facilitate the task, the first application is mad at the laboratory. The subsequent checks are carried out after seven days (T7), fourteen days (T14), twenty-one days (T21) and 28 days of treatment with E2 lotion and E3-A cream.

Following the above experimentation, the difference between the sebum-metric, corneous-metric and TEWL values obtained before and after the application of the products was evaluated using a polarised-light telecamera and mini DV, the variations of pimples and acneic pustules.

The sebum-metric, corneous-metric and TEWL values are then reported, processed and graphically displayed as shown in the following tables.

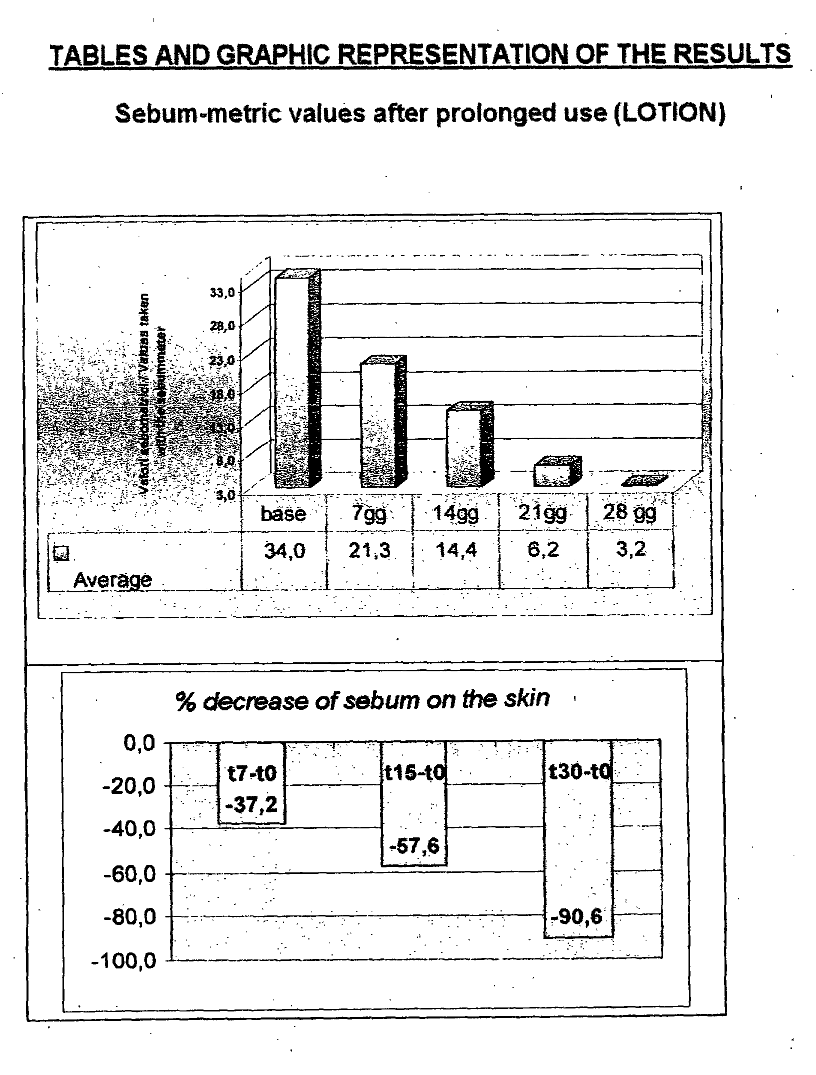

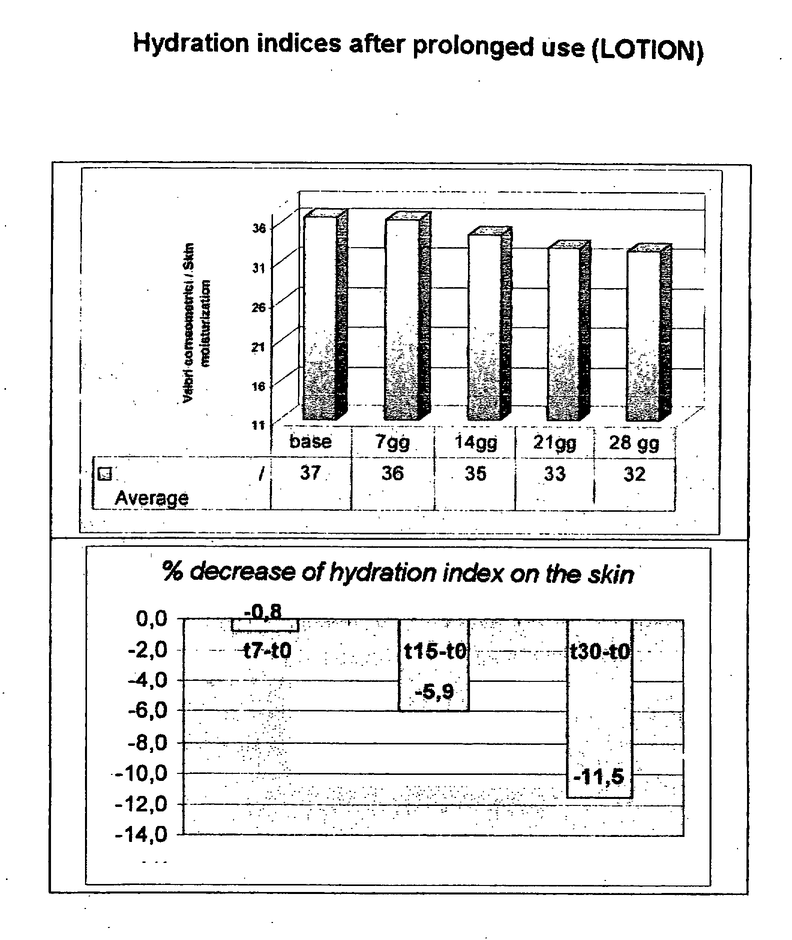

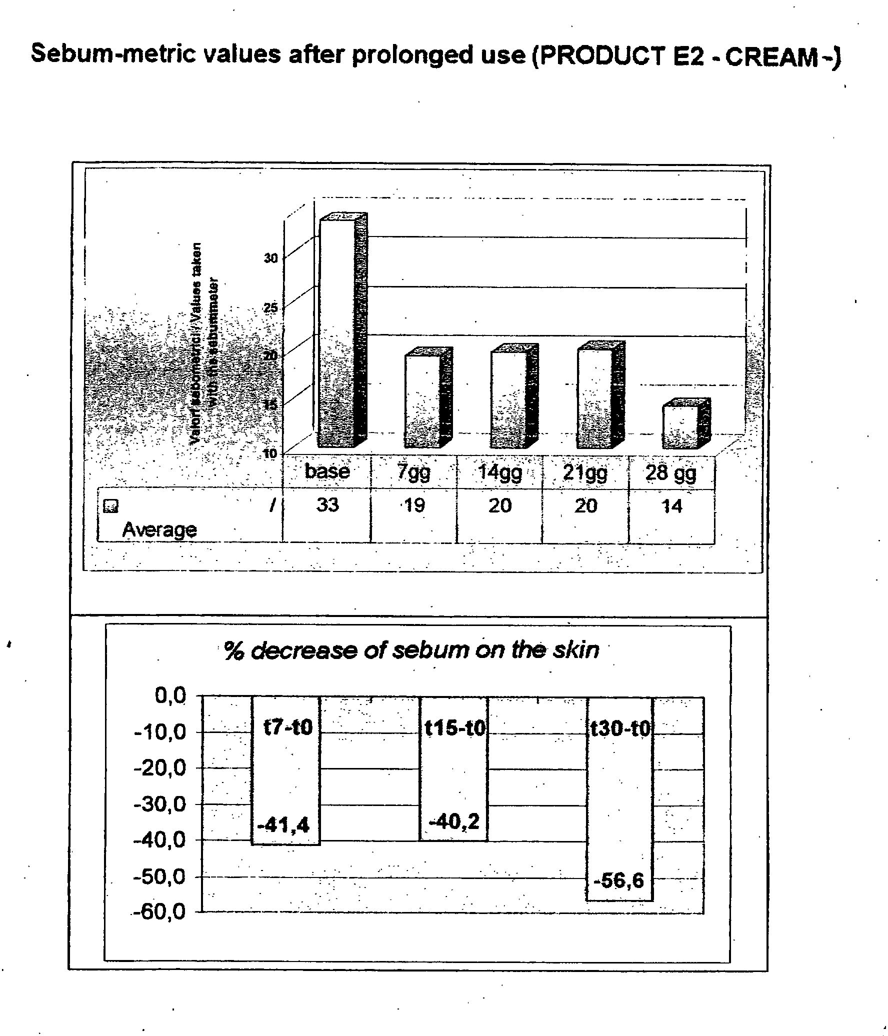

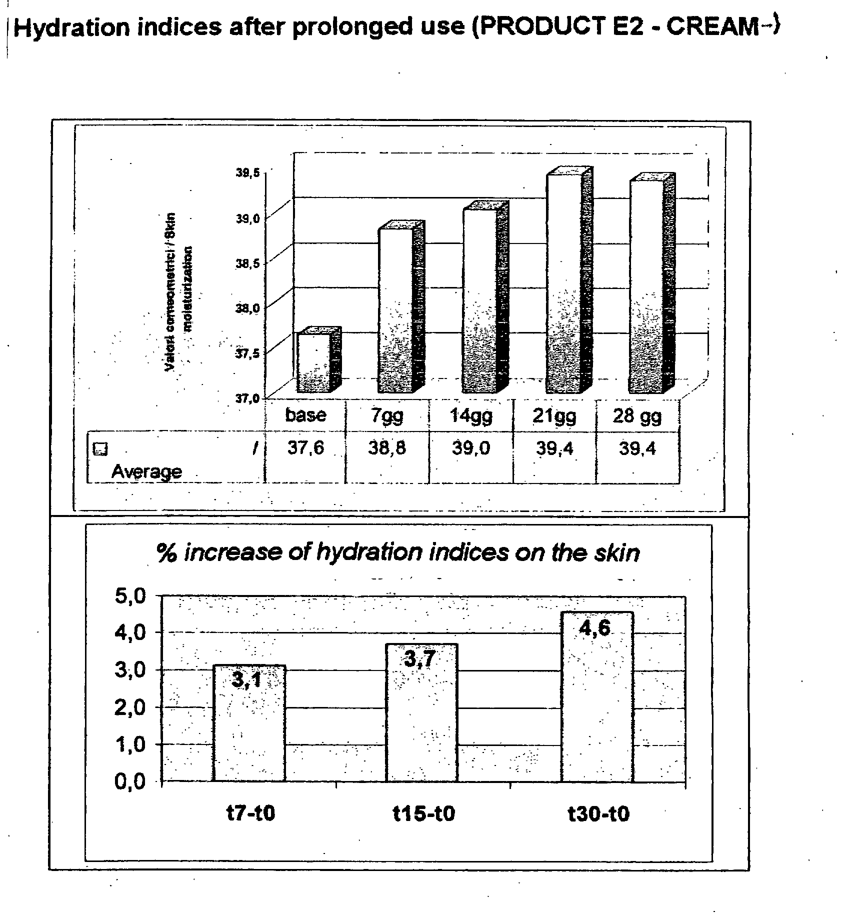

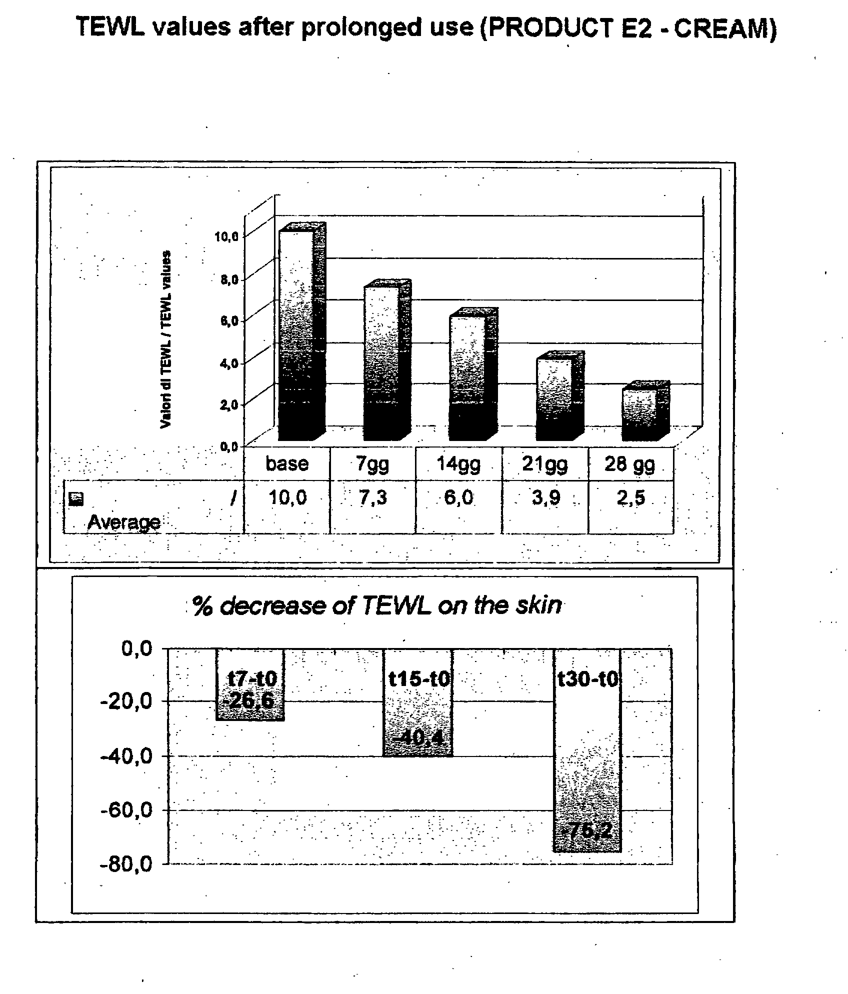

Below are some examples of formulations according to the present invention.

| Preparation 01 | | |
|---|---|---|
| No. | Description | % p/p a |
| 01 | Ethyl linoleate | 100.00 |
| How to prepare: | | |

| Preparation 02 | | |
|---|---|---|
| No. | Description | % p/p a |
| 01 | Triethyl citrate | 100.00 |
| How to prepare: | | |

| Preparation 03: | | |
|---|---|---|
| No. | Description | % p/p a |
| 01 | Ethyl linoleate | 20.00 |
| 02 | Triethyl citrate | 80.00 |
| How to prepare: mix 2 in 1 | | |

| Preparation 04: | | |
|---|---|---|
| No. | Description | % p/p a |
| 01 | Ethyl linoleate | 20.00 |
| 02 | Triethyl citrate | 20.00 |
| 03 | Salicylic acid | 2.00 |
| 04 | Ethylic alcohol | 58.00 |
| How to prepare: dilute 03 in 04, mix 02+03 to the resulting solution | | |

| Preparation 05: | | |
|---|---|---|
| No. | Description | % in weight |
| | Step A | |
| 01 | Ethyl linoleate | 5.00 |
| 02 | Ascorbic palmitate | 1.50 |
| 03 | Ppg-15 stearyl ether | 10.00 |
| 04 | Capryloyl glycine | 4.00 |
| 05 | Steareth-2 | 3.00 |
| 06 | Steareth-21 | 2.00 |

| | Step B | |
|---|---|---|
| 07 | Preservatives | as needed |
| 08 | Glycerol | 3.00 |
| 09 | Water as needed | 100 |
| How to prepare: step A) mix 01+02+03+04+05+06 and heat at +75°C. step B) Dilute the pre-mixed phase at +75°C and add by agitation to Step A). Cool at ambient temperature under agitation. | | |

| Preparation 06: | | |
|---|---|---|
| No. | Description | % p/p a |
| 01 | Ethyl linoleate | 20.00 |
| 02 | Triethyl citrate | 20.00 |
| 03 | Erythromycin | 10.00 |
| 04 | Ethylic alcohol | 50.00 |
| How to prepare: dilute 03 in 04, mix 02+03 to the resulting solution | | |

| Preparation 07: | | |
|---|---|---|
| No. | Description | % in weight |
| 01 | Trans-retinoic | 0.025 |
| 02 | Ethyl linoleate | 50.00 |
| 03 | Triethyl citrate | 20 |
| 04 | Ethylic alcohol as needed | 100 |
| How to prepare: dilute 01+02+03 in 04 | | |

| Preparation 08: | | |
|---|---|---|
| No. | Description | % in weight |
| 01 | Clindamycin | 1.00 |
| 02 | Ethyl linoleate | 5.00 |
| 03 | Triethyl citrate | 20 |
| 04 | Ethylic alcohol as needed | 100 |
| How to prepare: mix 02+03+04 and dilute 01 in the mixture | | |

| Preparation 09: | | |
|---|---|---|
| No. | Description | % in weight |
| 01 | Lipoic acid | 5 |
| 02 | Ethyl linoleate | 95 |
| How to prepare: dilute 01 in 02. | | |

It has thus been proved that the action of ethyl linoleate and/or of triethyl citrate, described in the treatment of acne, seborrhoea and oily skin, considering the particular mechanism of action of biological, pharmacological, physiological and biochemical type, is wider and is addressed to the treatment of several other cutaneous pathologies, such as athopic dermatitis, seborrhoeic dermatitis, nummular dermatitis, exfoliating dermatitis, stasis dermatitis, neuro-dermatitis, acne, acne rosacea, cicatricial alopecia, Hippocratic alopecia, female alopecia, medical toxic alopecia, aerate alopecia, pseudo-folliculitis, psoriasis, liche ruber planus, ichthyol, xerodermia, keratosis, decubitus ulcer, trophic ulcers, torpid sores, angioma, haemangioma, teleangectasy granuloma, seborrhoeic keratosis, etc.

Thanks to its peculiar mechanism of action at the skin level, the use of ethyl linoleate and/or triethyl citrate, optionally combined with suitable synergists, is innovative also as regards the cosmetic use, such as: anti-aging composition aimed at improving the aesthetical conditions of the skin, and at preventing skin aging signs, anti-wrinkle, hydrating, treatment of cutaneous hyper-pigmentation, cosmetic treatment of seborrhoeic skin, seborrhoeic skin prone to acne, etc.

The following examples of preparation are further illustrations of the composition of the present invention.

If not otherwise stated, the proportions therein are in percentage weight based on the final composition.

### Example of preparation 1

| Athopic dermatitis product | |
|---|---|
| Ingredients (A) | quantity by weight % |
| Steareth - 21 | 2.000 |
| Stearate glyceride | 3.000 |
| Cyclomethicon | 1.000 |
| PPG-15-stearyl ether | 2.000 |
| Beeswax | 2.000 |
| Stearic acid | 1.500 |
| Sunflower oil | 6.000 |
| Cetyl acid | 1.000 |
| Paraffin oil | 4.000 |
| Wheat grass oil | 1.000 |
| Ethyl linoleate | 7.500 |

| Ingredients (B) | quantity by weight % |
|---|---|
| Preservatives | as needed |
| Perfume | as needed |
| Water | as needed |
| How to prepare: Ingredients (A) and ingredients (B) are heated at 70° C separately. Ingredients (B) are then added to ingredients (A) mixing the whole to obtain a homogenised mixture in the form of emulsion for topical use. | |

### Example of preparation 2

| Product with skin acidifying activity | |
|---|---|
| Ingredients | |
| Triethyl citrate | 1.500 |
| Solubilizer | as needed |
| Water as needed | 100 |
| How to prepare: solubilize triethyl citrate in water using the solubilizer. | |

### Example of preparation 3

| Anti-acne product | |
|---|---|
| Ingredients (A) | quantity by weight % |
| Steareth - 21 | 2.000 |
| Stearate glyceride | 3.000 |
| Cyclomethicon | 1.000 |
| PPG-15-stearyl ether | 2.000 |
| Beeswax | 2.000 |
| Stearlc acid | 1.500 |
| Sunflower oil | 6.000 |
| Cetyl acid | 1.000 |
| Paraffin oil | 4.000 |
| Wheat grass oil | 1.000 |
| Ethyl linoleate | 7.500 |
| Triethyl citrate | 5.000 |

| Ingredients (B) | |
|---|---|
| Preservatives | as needed |
| Perfume | as needed |
| Water | as needed |
| How to prepare: Ingredients (A) and ingredients (B) are heated at 70° C separately. Ingredients (B) are then added to ingredients (A) mixing the whole to obtain a homogenised mixture in the form of emulsion for topical use. | |

### Example of preparation 4

| Athopic dermatitis product | |
|---|---|
| Ingredients (A) | quantity by weight % |
| Steareth - 21 | 2.000 |
| Stearate glyceride | 3.000 |
| Cyclomethicon | 1.000 |
| PPG-15-stearyl ether | 2.000 |
| Beeswax | 2.000 |
| Stearic acid | 1.500 |
| Sunflower oil | 6.000 |
| Cetyl acid | 1.000 |
| Paraffin oil | 4.000 |
| Wheat grass oil | 1.000 |
| Ethyl linoleate | 7.500 |
| Phosphatidylcoline | 0.050 |

| Ingredients (B) | |
|---|---|
| Preservatives | as needed |
| Perfume | as needed |
| Water | as needed |
| How to prepare: Ingredients (A) and ingredients (B) are heated at 70° C separately. Ingredients (B) are then added to ingredients (A) mixing the whole to obtain a homogenised mixture in the form of emulsion for topical use. | |

## Claims

1. Composition for topical use, in particular aimed at skin care and at improving the aesthetical conditions of the same, **characterised in that** as active ingredient, it contains at least one of the active principles selected between triethyl citrate and ethyl linoleate.

2. Composition for topical use according to claim 1, **characterised in that** ethyl linoleate is contained in a quantity between 0.001 and 90% weight/weight, preferably between 0.5 and 15% weight/weight.

3. Composition for topical use according to claim 1, **characterised in that** it contains, as active ingredient, triethyl citrate in a quantity between 0.001 and 90% weight/weight, preferably between 0.5 and 15% weight/weight.

4. Composition for topical use according to claim 1, **characterised in that** it contains, as active ingredients, ethyl linoleate and triethyl citrate, each in a quantity between 0.05 and 10% weight/weight, preferably in equi-molecular quantities.

5. Composition for topical use according to any one of the previous claims, **characterised by** a mechanism of action in which both ethyl linoleate and triethyl citrate behave as inert substances and are transformed into active principles when they come into contact with the skin, such transformation from inert substance to active principle, being a consequence of the hydrolysis that occurs due to specific cutaneous (esterase) or bacterial (lipase) enzymes, capable of releasing ethylic alcohol and, respectively, linoleic acid, diethyl citrate, mono-ethyl citrate and then, citric acid.

6. Composition for topical use according to the previous claims, **characterised in that** the bacterial lipases have a higher affinity to hydrolyse ethyl linoleate and triethyl citrate rather than the triglycerides contained in sebum.

7. Composition for topical use according to the previous claims, formulated for external use along with other active principles, such as acetic acid, lactic acid, salicylic acid, tartaric acid, glycolic acid, clindamycin, erythromycin, metronidazole, amoxicyllin, triclosan, capryloyl glycine, azelaic acid, hydroxide zinc, chloride zinc, retinol, trans-retinoic acid, resorcin, gentamicin, meclocycline, phenol, ascorbic acid, tocopherol, lipoic acid, phosphatidylcoline, phosphatidylserine, clorexidine, irgasan, phospholipids in general, in all dextrorotatory and laevorotatory forms, racemic mixtures, cis forms, trans forms and relevant salts, esters and amides, and formulations with particular additives and excipients for external use.

8. Composition for topical use according to the previous claims, **characterised in that** it also contains acetic acid, lactic acid, salicylic acid, tartaric acid, glycolic acid, clindamycin, erythromycin, metronidazole, amoxicyllin, triclosan, capryloyl glycine, azelaic acid, hydroxide zinc, chloride zinc, retinol, spironolactone, minocyclin, retinoic acid, retinol, retinaldehyde, resorcin, salicylic acid, phenol, ascorbic acid, tocopherol, lipoic acid, phosphatidylcoline, phosphatidylserine, phospholipids in general, in all dextrorotatory and laevorotatory forms, racemic mixtures, cis forms, trans forms, such substances being usable both individually and in combinations, in a quantity between 0.001 and 90% weight/weight, preferably in an overall quantity between 0.5 and 15% weight/weight.

9. Composition for topical use according to the previous claims, containing ethyl linoleate and/or triethyl citrate, optionally with other active ingredients and various additives, **characterised in that** it can be prepared in formulations for external use, such as emulsions of water in oil, emulsions of oil in water, mono-phase solutions, bi-phase pseudo-solutions, mono-phase gels, bi-phase gels. submicellar gels, anhydrous ointments, aspersory powders, alcoholates, alcoholites, alcoholic solutions, hydro-alcoholic solutions, and the like.
